# EUROPEAN PATENT APPLICATION

(11) **EP 3 871 660 A1**
(43) Date of publication of application: **01.09.2021**
(21) Application number: 19875830.2
(22) Date of filing: 24.10.2019
(51) Int. Cl.: A61K 9/00, A61K 9/08, A61K 31/375, A61K 31/7088, A61P 27/00, A61P 27/04

(54) **EYE DROP COMPOSITION COMPRISING APTAMIN C AS ACTIVE INGREDIENT**

(30) Priority: 25.10.2018 KR 20180128107
(71) Applicant: Nexmos Co., Ltd., Gyeonggi-do 16827 (KR)
(72) Inventor: SON, In Sik, Seongnam-Si Gyeonggi-do 13614 (KR)
(74) Representative: FRKelly
(86) International application number: PCT/KR2019/014045
(87) International publication number: WO 2020/085812

(57) **Abstract**

The invention relates to an eye drop composition comprising vitamin C and an aptamer that binds to the vitamin C as an active ingredient. the ophthalmic composition comprising aptamin C of the present invention maintains a low ROS level, and improves damages epithelial cells, and inflammatory response, so that it is effective in the improvement and treatment of dry eye syndrome with using the aptamer and antioxidant complex that maintains such an antioxidant effect for a long time.

## Description

### [Technical Field]

The present invention relates to an eye drop composition comprising aptamin C as an active ingredient.

### [Background Art]

In the modern society, stress on the cornea or conjunctiva is increasing due to wearing of contact lenses, techno stress, contact with allergies, air pollution, and the like, and accordingly, the number of people who suffer from dry eyes tends to increase. When dry eyes occur, the normal function of the cornea or conjunctiva decreases, and corneal epithelial disorders, corneal epithelial erosion, corneal ulcers, conjunctivitis, keratitis, eye infections, and the like are liable to occur.

Conventionally, a method of providing water retention by protecting the cornea or conjunctiva by instilling eye drops containing viscoelastic substances such as chondroitin sulfate and hyaluronic acid is known for the prevention or treatment of eye diseases that accompany dry eye or deterioration of the cornea or conjunctiva. However, with eye drops containing viscoelastic substances, the protective action on the cornea or conjunctiva is insufficient, and sufficient therapeutic effects may not be obtained, and development of new eye drops has been desired.

The condition of dry eye has not been completely clear, but it is known that dry eye causes cornea-conjunctival epithelial disorder and ultimately causes visual abnormalities. Therefore, it is very important to properly treat cornea-conjunctival epithelial disorders caused by dry eye.

Currently, eye drop treatment is the most common treatment for dry eye, and an eye drop containing sodium hyaluronate is widely used for dry eye treatment.

For eye drops containing sodium hyaluronate (hereinafter, simply referred to as "sodium hyaluronate eye drops"), it is intended to be used multiple times, and a multi-dose capable of freely opening and resealing caps, etc. Type (Hiarain (registered trademark) ophthalmic solution 0.1%, hyalin (registered trademark) ophthalmic solution 0.3%, etc.), and unit-dose type intended for one-time use (Hiarain (registered trademark) mini-ophthalmic solution 0.1%, hyalin (Registered trademark) mini eye drops 0.3%, etc.) exist.

In the case of multi-dose sodium hyaluronate eye drops, benzalkonium chloride is added as a preservative because the cap is opened and resealed, whereas the unit-dose type is used once (one-time use) is not added.

Preservatives such as benzalkonium chloride have a risk of causing corneal disorders. For example, It is known that benzalkonium chloride is a possibility to cause a concentration-dependent corneal epithelial disorder as disclosed in Clinical and Experimental Ophthalmology, 32, 180-184 (2004). As described above, since dry eye is originally a disease accompanying corneal epithelial disorders, unit-dose sodium hyaluronate eye drops are used for patients with severe dry eye. On the other hand, it is difficult to prescribe unit-dose sodium hyaluronate eye drops to all dry eye patients due to production cost problems, so the risk of corneal epithelial disorders is reduced by reducing the preservative concentration in multi-dose eye drops. It is also considered. However, in practice, if the concentration of the preservative in the eye drops is reduced, a sufficient preservation effect for use as a multi-dose type cannot be obtained, and the physicochemical properties of the eye drops are conventionally changed due to the change of the blending components due to the reduction of the preservative It is also a concern that it will be different from the sodium hyaluronate eye drops.

### [Prior patent literature]

International Patent Publication No. WO 2002/22131

### [Disclosure]

### [Technical Problem]

The present invention solves the above problems and is due to the necessity of the above, and an object of the present invention is to provide an eye drop composition for treating dry eye syndrome.

### [Technical Solution]

In order to achieve the above object, the present invention provides an eye drop composition comprising vitamin C and an aptamer that binds to the vitamin C as an active ingredient.

In one embodiment of the present invention, the aptamer binding to vitamin C is preferably composed of the nucleotide sequence of SEQ ID NO: 1 (AGAGCTCGCGCCGGAGTTCTCAATGCAAGAGC), but all aptamers that can achieve the object of the present invention are included within the protection scope of the present invention.

In one embodiment of the present invention, the composition molar ratio of the vitamin C and the aptamer binding to the vitamin C is preferably in the range of 10000:1 to 10:1, but is not limited thereto.
Hereinafter, the present invention will be described.

Oxidative stress signaling by ROS plays a key role in promoting the dry eye cycle by causing damage and inflammatory reactions to epithelial cells and goblet cells on the surface of the eyeball. Therefore, it is necessary to maintain a low ROS level in order to reduce excessive oxidative stress signaling..

In the present invention, since the use of an antioxidant (ex. Vitamin C) maintains a low ROS level and improves epithelial cell damage and inflammatory response, it was confirmed that the use of an aptamer and antioxidant complex that maintains the antioxidant effect of the present invention for a long time is effective in improvement and treatment of dry eye syndrome.

In preparing the eye drops of the present invention, in addition to the aptamin C complex of the present invention described above, if necessary, Various pharmaceutically acceptable additives like isotonic agents such as sodium chloride, potassium chloride, calcium chloride, glycerin, and propylene glycol, buffers such as ε-aminocaproic acid boric acid, citric acid, disodium hydrogen phosphate, preservatives such as benzalkonium chloride, chlorohexidine gluconate, benzethonium chloride, sorbic acid, potassium sorbate, ethyl paraoxybenzoate, butyl paraoxybenzoate, etc. can be blended.

The pH of this eye drop solution is preferably 3 to 8, particularly 4 to 7.

The preparation method of the eye drop solution of the present invention does not require any special method or operation, and/or can be prepared by a method commonly used.

### [Advantageous Effects]

As can be seen through the present invention, the ophthalmic composition comprising aptamin C of the present invention maintains a low ROS level, and improves damages epithelial cells, and inflammatory response, so that it is effective in the improvement and treatment of dry eye syndrome with using the aptamer and antioxidant complex that maintains such an antioxidant effect for a long time.

### [Description of Drawings]

Fig. 1 is a graph showing the maintenance of the reducing power of vitamin C by aptamin C, a graph showing the results of the OPDA (12-oxo-phytodienoic acid) assay (5 mM Vitamin C, 1 µM aptamin C).

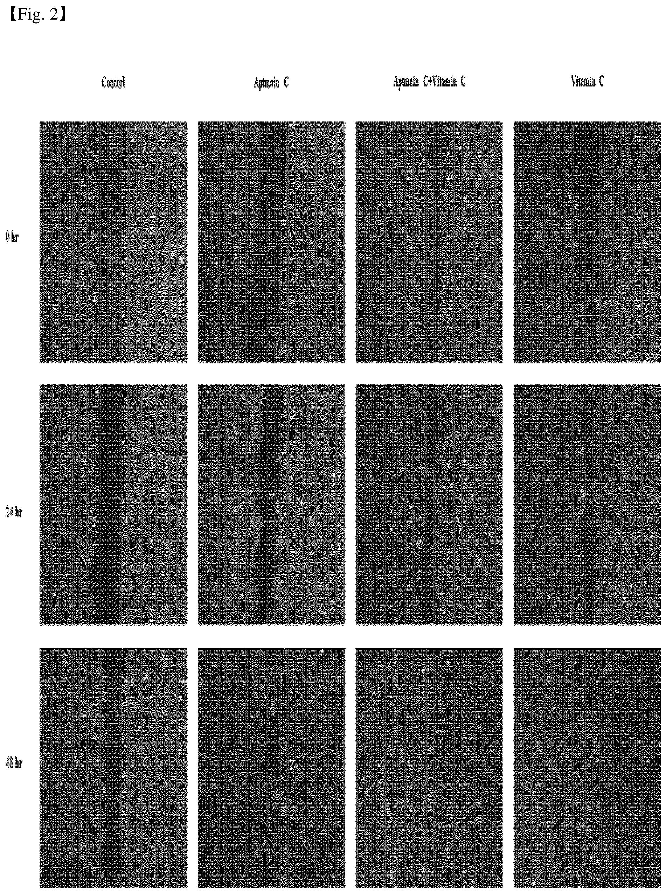

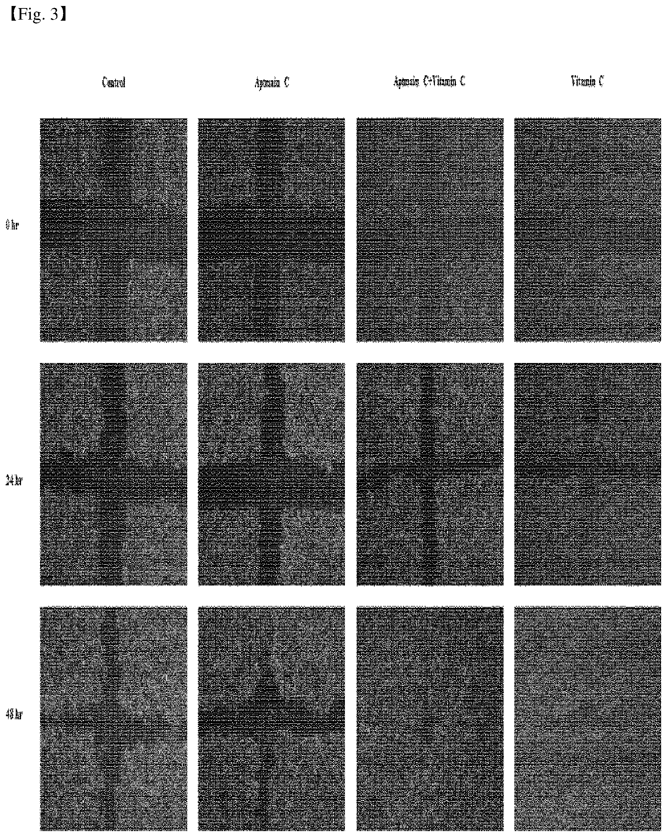

Figures 2 and 3 are results of the wound healing assay in human keratinocyte (HaCaT cell line), when treated with vitamin C and aptamin C (aptamer binding to vitamin C), a representative picture showing the degree of healing over time when vitamin C and aptamin C (aptamer binding to vitamin C) were treated.

In this experiment, the efficacy was confirmed by treating aptamin C with 1 µM (0.001%) and vitamin C with 0.5 mM (0.008%). As a result of this experiment, it was confirmed that the wound healing was best when the composition containing aptamin C and vitamin C was treated.

In this figure and invention, the aptamer binding to vitamin C (ascorbic acid) was named aptamin C.

### [Mode for Invention]

### Example 1: Preparation of the composition of the present invention

The composition of the present invention was prepared by mixing the synthesized aptamer and vitamin C (DSM, UK) effective in Example 1 in the weight ratio described in distilled water at room temperature.

For example, 0.008% (w/v) of ascorbic acid and 0.001% (w/v) of aptamin C bound to vitamin C were mixed in distilled water to prepare an eye drop composition of the present invention.

### Example 2: Maintaining vitamin C reducing power through aptamer

After heating the aptamin C dissolved in the annealing buffer to 95°C, gradually lowering the temperature to room temperature to create the secondary structure of the aptamer, and then mix it with the reduced L-ascorbic acid so that the aptamer can bind to L-ascorbic acid. It was reacted for about 30 minutes. Thereafter, hydrogen peroxide solution was added to create oxidation conditions, and the oxidation of L-ascorbic acid was measured by adding a fluorescent dye, OPDA (o-phenylenediamine). The degree of DHA production can be quantitatively analyzed by measuring the amount of fluorescence from DHA-OPDA generated by reacting DHA, an oxidative product of L-ascorbic acid, with OPDA. In the above conditions, the amount of fluorescence of DHA-OPDA was measured every 2 minutes for 12 hours.

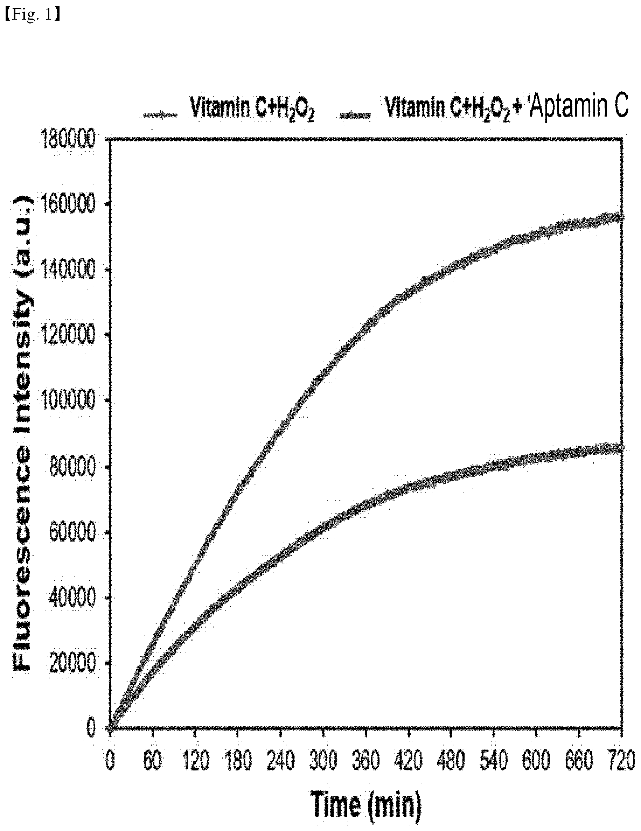

As can be seen in FIG. 1, as a result of the OPDA assay, when vitamin C and aptamin C were treated together, the result was obtained that the oxidation of vitamin C was prevented, so that an eye drop composition comprising Vitamin C and an aptamer that specifically binds to vitamin C as an active ingredient is expected to slow the oxidation rate of Vitamin C to maintain the therapeutic efficacy for dry eye.

### Example 3: wound healing assay in human keratinocyte (HaCaT cell line)

Seeding human keratinocytes (HaCaT cell cells) [obtained at Sookmyung Women's University Life Systems Research Institute, South Korea] (1 × 10⁵ cells/ml) in a 6-well plate at 500 µL/well, and incubated in DMEM 95% air in 5% CO2, 37°C.

The cells were wounded by scratching the surface of the culture with a 200 µL pipette tip.

The scratched HaCaT cells were washed with fresh medium to remove non-adherent cells.

1 µM (0.001%) of aptamin C, 0.5 mM (0.008%) of vitamin C, and a mixture of aptamin C and vitamin C were each treated in a medium and cultured for 24 hours.

The cell migration was measured using an inverted phase contrast light source microscope.

The damaged area of each well was photographed and the area was measured using Image J.

The average area between the edges of the migrated cells was determined using Image J.

The percentage of injured suturing by HaCaT cells was compared for the aptamin C and vitamin C treatment alone, and for a composition in which aptamin C and vitamin C were mixed.

## Claims

1. An eye drop composition comprising vitamin C and an aptamer that binds to the vitamin C as an active ingredient.

2. The eye drop composition according to claim 1, wherein the aptamer binding to vitamin C is composed of the nucleotide sequence of SEQ ID NO: 1.

3. The eye drop composition according to claim 1, wherein the composition molar ratio of the vitamin C and the aptamer binding to the vitamin C is in the range of 10000:1 to 10:1.


<110>    Nexmos Co., Ltd.

<120>    AN OPHTHALMIC SOLUTION CONTAINING APTAMIN C AS  EFFECTIVE
         COMPONENT

<130>    OP19-0030HSPCT

<150>    KR 10-2018-0128107
<151>    2018-10-25

<160>    1

<170>    KopatentIn 2.0

<210>    1
<211>    32
<212>    DNA
<213>    Artificial Sequence

<220>
<223>    aptamer


<400>    1
agagctcgcg ccggagttct caatgcaaga gc                                32